## Europäisches Patentamt

## European Patent Office

## Office européen des brevets

(11) Veröffentlichungsnummer: **0 035 173**
**B1**

(12) **EUROPÄISCHE PATENTSCHRIFT**

(45) Veröffentlichungstag der Patentschrift:
**10.08.83**

(21) Anmeldenummer: **81101163.4**

(22) Anmeldetag: **19.02.81**

(51) Int. Cl.³: **B 01 J 19/00,** C 07 C 51/265,
C 07 C 51/215

(54) Anordnung zum Übertragen der Wärme und zum Ausgleichen der Wärmeprofile in Anlagen zur Herstellung von Phthalsäureanhydrid und Maleinsäureanhydrid.

(30) Priorität: **29.02.80 DE 3007627**

(43) Veröffentlichungstag der Anmeldung:
**09.09.81 Patentblatt 81/36**

(45) Bekanntmachung des Hinweises auf die Patenterteilung:
**10.08.83 Patentblatt 83/32**

(84) Benannte Vertragsstaaten:
**AT BE DE FR GB IT**

(56) Entgegenhaltungen:
**DE-A-2 548 293**
**FR-A-1 146 163**
**GB-A-1 130 330**

(73) Patentinhaber: **BASF Aktiengesellschaft,
Carl-Bosch-Strasse 38, D-6700 Ludwigshafen (DE)**

(72) Erfinder: **Muff, Wolf, Von-Goethe-Strasse 10,
D-6711 Laumersheim (DE)**

ACTORUM AG

Anordnung zum Übertragen der Wärme und zum Ausgleichen der Wärmeprofile in Anlagen zur Herstellung von Phthalsäureanhydrid und Maleinsäureanhydrid

Die Erfindung betrifft eine Anordnung zum Übertragen der Wärme und zum Ausgleichen der Wärmeprofile für Anlagen zur Herstellung von Phthalsäureanhydrid (PSA) und Maleinsäureanhydrid (MSA) mit mindestens einem Heiz- und Kühlkreis zum zyklisch alternierenden Heizen und Kühlen mehrerer Produktabscheider.

Es sind bereits Verfahren zur Wärmerückgewinnung der bei Gasphasenoxidation von Kohlenwasserstoffen in einem Rohrbündelwärmeaustauscher mit auf der Mantelseite umlaufendem flüssigen Wärmeträger bekannt, bei denen man mittels des Wärmeträgers mit einem Teil der Reaktionswärme Dampf erzeugt und die zeitlich sehr ungleichmässig abfallende restliche Reaktionswärme dazu verwendet, den aus der Teilwärmemenge erzeugten Dampf zu überhitzen. – Ein optimaler Ausgleich der unterschiedlichen Wärmeprofile zwischen den Heiz- und Kühlphasen ist indes mit einer derartigen Anordnung zur Wärmeverwertung nicht erreichbar.

Bei der bisher üblichen Ausführung des Wärmeträgerkreislaufes zum Kühlen und Aufheizen der PSA-Abscheider tritt periodisch ein sehr hoher Spitzenbedarf an Heizdampf auf. Dieser Spitzenbedarf wird im allgemeinen aus vorhandenen Dampfnetzen des Herstellerbetriebes gedeckt, obwohl das gesamte Wärmeaufkommen aus der exothermen Reaktion einen Überschuss ausweist. Dieser Nachteil bzw. Mangel ist insbesondere bei kleineren Anlagen zur Herstellung von Phthalsäureanhydrid und Maleinsäureanhydrid gegeben, bei denen ein weitgehender Ausgleich der Wärmeprofile wegen Fehlens eines grösseren Dampfnetzes zur Wärmeaufnahme und -abgabe nicht erreicht werden kann.

Der Erfindung liegt demzufolge die Aufgabe zugrunde, die bekannten Wärmeträgerkreisläufe, also den Kaltölkreislauf für die Kühlphasen des Abscheiders bzw. mehrerer Abscheider einerseits und den Heissölkreislauf zum Abschmelzen des niedergeschlagenen Produktes andererseits so zu verändern, bzw. zu ergänzen, dass sich wesentlich vergleichmässigte Wärmeprofile ergeben und die überschüssig anfallende exotherme Abwärme wirtschaftlich besser eingesetzt werden kann.

Erfindungsgemäss wird diese Aufgabe dadurch gelöst, dass
- dem Heizkreis mit vorgegebener Temperatur ein Speicherkreis mit höherer Temperatur überlagert ist, durch den ein Wärmeträger zwangsweise gefördert wird,
- im Speicherkreis und im Heizkreis der gleiche Wärmeträger eingesetzt wird,
- die Wärme aus dem Speicherkreis auf den Heizkreis durch Mischen übertragen wird.

Die Zeichnungen zeigen die erfindungsgemässe Anordnung der verknüpften Kreisläufe in Fig. 1 und weisen in den Diagrammen nach Fig. 2 und 3 die Vorteile aus, die sich bei Anwendung der Erfindung ergeben.

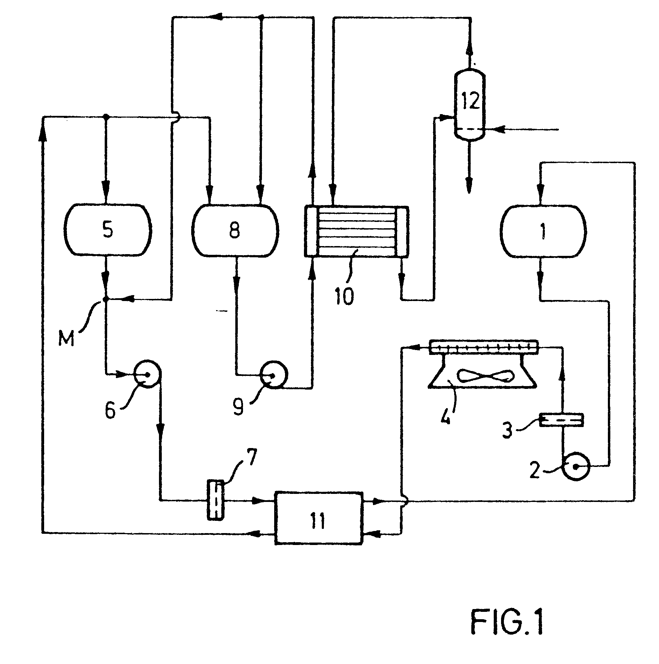

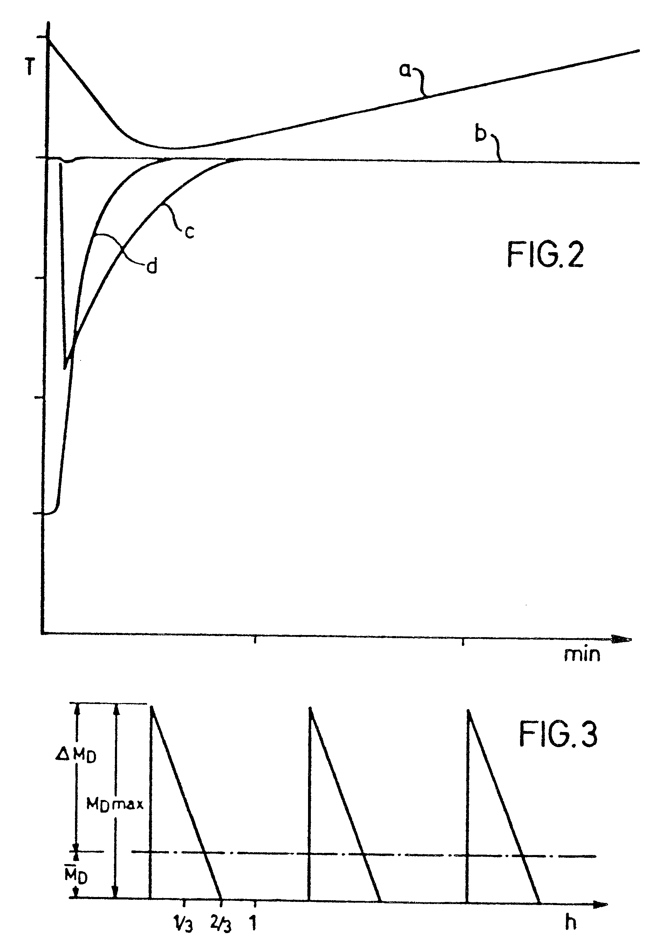

Das erfindungsgemässe und für eine Anlage zur Herstellung von Phthalsäureanhydrid beschriebene Wärmeträgersystem besteht gemäss Fig. 1 aus drei Kreisläufen verschiedenen Temperaturniveaus. Der sog. Kaltölkreislauf ist gebildet aus dem Kaltölbehälter 1, der Umwälzpumpe 2, dem Ölfilter 3 und dem Ölkühler 4 zum Kühlen eines oder mehrerer Produktabscheider 11. Der zum zeitweisen Aufheizen der Produktabscheider 11, – also zum Abschmelzen des sublimierten Produktes – gebildete Heissölkreislauf (Heizkreis) besteht aus dem Heizölbehälter 5, der Umwälzpumpe 6 und dem Ölfilter 7. Die Produktabscheider 11 für PSA-Anlagen sind Mehrweg-Wärmetauscher, deren Betriebsweise mit aufeinanderfolgenden Heiz- und Kühlphasen an dieser Stelle als bekannt vorausgesetzt werden kann. – In Fig. 3 ist für eine bestimmte Anlage die zeitliche Aufeinanderfolge der Heizphasen der Abscheider 11 mit sägezahnförmigen Linienzügen idealisiert schematisch wiedergegeben.

Der Speicherkreislauf besteht aus dem Wärmespeicher 8, der Umwälzpumpe 9 und dem Aufheizer 10. Das Übertragen der Wärme an den Heissölkreislauf erfolgt am Mischpunkt M. Als Wärmeträger im Speicherkreislauf wird zweckmässig das gleiche Medium wie im Heizkreislauf eingesetzt, im vorliegenden Fall z.B. ein Gemisch aus Diphenyl und Diphenyloxid, nachfolgend «Öl» genannt. Dieses Öl wird im Aufheizer 10, der als Zweiwegwärmetauscher gestaltet ist, aus einem vorhandenen Dampfnetz auf eine Temperatur gebracht, die höher liegt als die des gleichen Wärmeträgers im Heissölkreislauf über den Behälter 5 und die Umwälzpumpe 6. Die Verknüpfung des Speicherkreislaufs mit dem Dampfnetz erfolgt am Apparat 12. Das ist vorzugsweise ein dampfgesättigter Kondensatsammelbehälter, in den der überschüssige Dampf aus der exotherm ablaufenden Reaktion, durch zurücklaufendes Kondensat gesättigt und danach dem Aufheizer 10 zugeführt wird.

Mittels des Heissölkreislaufs wird in den Heizphasen den Abscheidern 11 die erforderliche Wärme zum Abschmelzen des ansublimierten Produktes zugeführt. Zu diesem Zweck wird heisses Öl – aufgeheizt über den Speicherkreislauf – mit der Pumpe 6 aus dem Behälter 5 in einen oder mehrere der Abscheider 11 gepumpt. Das Öl kühlt dabei ab und muss deshalb durch Zufuhr von heisserem Öl aus dem Speicher 8 wieder auf höhere Temperatur gebracht werden. Die Zufuhr von heisserem Öl aus dem Speicher 8 wird über die Temperatur des Heissölvorlaufs selbsttätig geregelt. Der Flüssigkeitsstand im Speicher 8 wird durch weitere Zufuhr des Wärmeträgers aus dem Rücklauf des Heissölkreislaufs konstant gehalten. Dabei kühlt der Speicher 8 allmählich ab.

Nach einer gewissen Zeit haben die Abscheider 11 annähernd die Temperatur des Heissöls erreicht und es muss kein weiteres heisses Öl aus dem Speicher 8 mehr zugeführt werden. Zu diesem Zeitpunkt hat auch die Temperatur des Wärmeträgers im Speicherkreislauf ihren niedrigsten Wert erreicht. Für den Rest des Abschmelzvorganges des Produkts wird nur noch so viel heisses Öl am Mischpunkt M zugegeben, wie erforderlich ist, um die Wärmeverluste zu decken. In dieser Zeit wird der Inhalt des Speichers 8 wieder auf seine ursprüngliche Temperatur aufgeheizt. Dazu wird mit der Pumpe 9 der Inhalt des Speichers 8 ständig über den Aufheizer 10 umgewälzt. Die dem Aufheizer 10 zuzuführende Heizdampfmenge wird so geregelt, dass sie über dem gesamten Zyklus gleich ist. Sie entspricht dann dem durchschnittlichen Wärmebedarf des Heissölsystems.

Der Temperaturverlauf im Speicher 8, im Heissölvorlauf und Heissölrücklauf sind in Fig. 2 schematisch dargestellt. Die Kurve a gibt den zeitlichen Verlauf im Speicher 8 für einen Zyklus wieder; die Temperatur des Heissölvorlaufs zum Abscheider 11 ist in Fig. 2 mit b bezeichnet. – Die Temperatur im Behälter 5 fällt zu Beginn des Abschmelzvorganges in den Abscheidern 11 auf einen extrem niedrigen Wert und steigt dann mit dem Verlauf der Kurve c bis auf den Wert der Heissölvorlauftemperatur gemäss Linienzug b an. Der zeitliche Verlauf der Temperatur des Heissölrücklaufs vom Abscheider 11 zum Behälter 5 ist mit dem Linienzug d wiedergegeben.

Voraussetzung für die Funktion des oben beschriebenen Systems von drei verknüpften Kreisläufen ist, dass der Speicherinhalt und die Speichertemperatur des Speicherkreislaufs und die Dampfmenge sorgfältig aufeinander abgestimmt sind. Es ergeben sich dann folgende wesentliche Vorteile:

1. Eine Glättung des Dampfbedarfs und dadurch die Möglichkeit einer sinnvolleren Nutzung des Überschussdampfs auch beim Nichtvorhandensein eines grösseren Dampfnetzes. (Siehe nachfolgendes Beispiel 1).
2. Eine wesentliche Verkürzung der Aufheizzeit und damit eine längere Nutzungszeit der Abscheider.
3. Die Möglichkeit, von Zeit zu Zeit die Temperatur des Öls auf 250°C anzuheben, um dadurch korrosive Substanzen auf den Rohrbündeln der Abscheider 11 zu zersetzen. Auf diese Weise kann die Lebensdauer der Abscheider wesentlich erhöht werden.

Beispiel 1

In einer Anlage zur Herstellung von Phthalsäureanhydrid sind vier Abscheider eingesetzt. Ein Zyklus läuft wie folgt ab: Die Beladung der Apparate (Kühlphase) erfolgt in 4,5 Stunden. Danach werden die Abscheider 40 Minuten lang auf 190°C aufgeheizt und weitere 20 Minuten lang bei 190°C gehalten, um das sublimierte Produkt abzuschmelzen, danach werden die Abscheider 30 Minuten lang zurückgekühlt. Der Wärmebedarf eines Zyklus beträgt 2 512 000 kJ. Zur Vereinfachung sind hierbei Isolationsverluste nicht berücksichtigt. Im Vergleich mit den bisher üblichen Verfahren ohne Speicherkreislauf ergeben sich die in Fig. 3 graphisch dargestellten Verhältnisse. Der in Anlagen nach dem Stand der Technik gegebene zeitliche Verlauf des Dampfverbrauchs ist in ausgezogenen Linien dargestellt. Der zeitliche Verlauf des Dampfverbrauchs mit einem richtig angepassten Speichersystem ist in der gleichen Anlage konstant, wie es die strichpunktierte Linie in Fig. 3 wiedergibt. Es bedeuten ferner

$M_{D\,max}$ = Maximale Dampfmenge, die vorgehalten werden muss, um den Abscheider in 49 Minuten auf 200°C zu erhitzen, im Beispiel 4,5 t/h.
$\overline{M}_D$ = Dampfverbrauch bei Einbau eines Speichersystems, im Beispiel ca. 1 t/h.
$\Delta M_D$ = Zusätzlich für Turbinen oder andere kontinuierlich arbeitende Dampfabnehmer nutzbarer Dampf, im Beispiel 3,5 t/h.

Beispiel 2

Ebenso wie im Beispiel 1 beträgt die Aufheizzeit bis zum Erreichen der Haltetemperatur von 190°C etwa 40 Minuten. Diese Zeit ist durch die maximal zur Verfügung stehende Dampfmenge begrenzt. – Die minimal mögliche Aufheizzeit wird dagegen lediglich durch den Wärmeübergang zwischen dem Wärmeträger Öl und den Abscheidern 11 festgelegt. Wird die Pumpe 9 im Speicherkreislauf so bemessen, dass am Mischpunkt M mindestens so viel Wärme aus dem Speicherkreislauf in den Heizkreislauf übertragen wird, wie vom Heizkreislauf an die Abscheider 11 übertragen werden kann, so können bei Einsatz eines Speichers 8 gemäss Fig. 1 die tatsächliche und die minimal mögliche Aufheizzeit in Einklang gebracht und die Aufheizzeit damit entscheidend verkürzt werden.

Beispiel 3 (ohne Speicherung)

| | |
|---|---|
| Beladungszeit ≙ nutzbarer Zeit | 270 Min. |
| Aufheizzeit | 40 Min. |
| Haltezeit | 20 Min. |
| Rückkühlzeit | 30 Min. |
| Zykluszeit | 360 Min. |
| davon nutzbar | 75 % |

Beispiel 4 (mit Speicher)

| | |
|---|---|
| Beladungszeit ≙ nutzbarer Zeit | 270 Min. |
| Aufheizzeit | 12 Min. |
| Haltezeit | 20 Min. |
| Rückkühlzeit | 30 Min. |
| Zykluszeit | 332 Min. |
| davon nutzbar | 81 % |

Die Aufheizzeit lässt sich also um 46% verkürzen. Damit lässt sich also entsprechend diesem Beispiel die nutzbare Zeit der Apparate um ca. 8% verlängern.

**Patentanspruch**

Anordnung zum Übertragen der Wärme und zum Ausgleichen der Wärmeprofile für Anlagen zur Herstellung von Phthalsäureanhydrid und Maleinsäureanhydrid mit mindestens einem Heiz- und Kühlkreis zum zyklisch alternierenden Heizen und Kühlen mehrerer Produktabscheider, dadurch gekennzeichnet, dass

– dem Heizkreis mit vorgegebener Temperatur ein Speicherkreis mit höherer Temperatur überlagert ist, durch den ein Wärmeträger zwangsweise gefördert wird,
– im Speicherkreis und im Heizkreis der gleiche Wärmeträger eingesetzt wird,
– die Wärme aus dem Speicherkreis auf den Heizkreis durch Mischen übertragen wird.

## Claim

Arrangement for transferring heat and levelling out temperature profiles for plants for the production of phthalic anhydride and maleic anhydride, comprising one or more heating and cooling circuits for the cyclically alternating heating and cooling of a plurality of product separators, wherein
– the heating circuit, operating at a predetermined temperature, has superposed on it a storage circuit which is at a higher temperature and through which a heat transfer medium is forcibly conveyed,
– the same heat transfer medium is employed in the storage and heating circuits, and
– the heat from the storage circuit is transferred to the heating circuit by mixing.

## Revendication

Agencement pour la transmission de la chaleur et l'égalisation des profils thermiques dans des installations de préparation d'anhydride phtalique et d'anhydride maléique, comportant au moins un circuit de chauffage et de refroidissement pour réchauffer et refroidir alternativement, de façon cyclique, une pluralité de séparateurs de produit, caractérisé par le fait que
– au circuit de chauffage à température prédéterminé, est superposé un circuit accumulateur à température plus élevée, qui assure l'alimentation forcée d'un véhicule de chaleur,
– c'est le même véhicule de chaleur qui est introduit dans le circuit accumulateur et dans le circuit de chauffage
– la chaleur prélevée du circuit accumulateur est transmise par mélange au circuit de chauffage.

FIG.1

FIG.2

FIG.3